# EUROPEAN PATENT SPECIFICATION

(11) **EP 1 684 631 B1**
(45) Date of publication and mention of the grant of the patent: **08.04.2009**
(21) Application number: 04797796.2
(22) Date of filing: 08.11.2004
(51) Int. Cl.: A61B 5/103

(54) **USE OF A SYSTEM FOR CHARACTERIZING TACTILE PERCEPTION**
VERWENDUNG EINES SYSTEMS ZUR CHARAKTERISIERUNG DER TAKTILEN WAHRNEHMUNG
UTILISATION D'UN SYSTEME DE CARACTERISATION DE LA PERCEPTION TACTILE

(30) Priority: 13.11.2003 US 712490
(43) Date of publication of application: 02.08.2006
(73) Proprietor: Unilever PLC, London EC4Y 0DY (GB); Unilever N.V., 3013 AL Rotterdam (NL)
(72) Inventor: DUSSAUD, Anne, Unilever Home & Personal Care, Trumbull, Connecticut 06611 (US); DING, Junqi, Unilever Home & Personal Care USA, Trumbull, Connecticut 06611 (US); LIPS, Alexander, Unilever R & D Edgewater, Edgewater, New Jersey 07020 (US)
(74) Representative: Acham, Nicholas Clive
(86) International application number: PCT/EP2004/012751
(87) International publication number: WO 2005/046474

(56) References cited:
- WO-A1-03/001865
- WO-A2-02/24071
- FR-A1- 2 811 764
- US-A- 5 588 440
- OMATA S: "NEW TYPE TACTILE SENSOR FOR SENSING HARDNESS LIKE THE HUMAN HAND AND ITS APPLICATIONS FOR LIVING TISSUE" TECHNICAL DIGEST OF THE SENSOR SYMPOSIUM, XX, XX, 1990, pages 257-260, XP000609280

## Description

### Field of the Invention

The invention concerns a method of using a system for demonstrating proof of efficacy and/or facilitating cosmetic product selection.

### Background of the Invention

The ultimate goal of any cosmetic product or method, is a satisfied consumer. Many cosmetic products, either leave-on and/or wash off products, advertise a variety of skin benefits. While expert graders may be trained to use diagnostic equipment or to perceive the difference, consumers usually cannot easily discern whether the claimed benefit is actually delivered, or a quantitative extent to which it is delivered. Skin conditions are typically evaluated subjectively through the senses, particularly through sight and touch. An objective measure of the physical parameters controlling the key attributes would provide a useful tool in the characterization of sensory attributes.

Among skin diagnostic techniques, acoustic emission has not been commonly used for skin characterization. Acoustic emission has been commonly used in the field of mechanical systems and musical instruments. It has also been used in the field of fabric sensory, since for consumers, the sound of fabrics is part of the sensory experience.

Tanaka, M. et al., "The 'Haptic Finger' - a new device for monitoring skin condition," Skin Research and Technology, 9:131-136 (2003) describe a device simulating a finger, formed of several layers of materials (stainless steel plate, sponge rubber, a piezopolymer film, acetate film and gauze). This artificial finger is rubbed on skin as it records the mechanical stresses generated in flexible piezopolymer films.

Flament, F., et al., "Finger perception metrology. Correlation between friction force and acoustic emission, " Abstract of a presentation at a skin conference in Hamburg, 2003, describe a method where the finger of a subject is held by a motorized support which moves the finger over a surface of materials where the friction force and the acoustic emission signals are detected.

WO 02/24071 A2 relates to a method, apparatus and system for assessing hair condition by measuring friction in hair samples. A friction member, similar to a "comb" generates friction at the contact between the comb and the hair. The frictional noise generated by the forces during comb motion is captured by a frictional noise sensor.

A need remains for a tool and method for the objective measurement of tactile attributes of human skin.

### Summary of the Invention

Accordingly, Applicants have developed a method employing an acoustic emissions system for objective assessment of skin condition before, during, and after application and/or wash-off of products onto or from skin. The acoustic emission measurement system includes:
(A) means for detecting an acoustic emission signal generated from skin/skin contact;
(B) means for collecting, storing and displaying said emission signal; and
(C) means for correlating said emission signal with an attribute of the skin.

The system is used as a clinical tool to evaluate the efficacy of cosmetic skin care and/or cleansing products wherein the acoustic emission signal is generated from the skin on one area of the human body sliding on the skin of another area of the body.

The very sensitive inventive method detects acoustic signals during touching to assess the in-use sensory performance of personal care products and extract specific sensory attributes or sensory profile. The system and method can be used to support claims about various benefits from skin care products, such as moisturization, rinsability of cleansers and a wide range of sensory benefits (i.e smoothness, silkiness).

### Brief Description of the Drawings

Although not limited thereto, additional objects, features and benefits of the present invention will become more readily apparent from consideration of the drawings in which:
Fig.1 is a schematic diagram of an acoustic emission system according to the present invention;
Fig. 2 shows acoustic profiles of sodium PEG lauryl ether sulphate (SLES)/water solution at three different concentrations (2A = 1% w/w; 2B = 0.2% w/w; and 2C = 0.01% w/w), generated using the system of FIG. 1;
Fig. 3 shows an acoustic profile of a wash-off (cleansing) product obtained with the system of FIG. 1, wherein a hydrophone and accelerometers are used simultaneously;
Fig. 4 shows acoustic profiles of two different cleansing products (products A and B) obtained using the same system as that used to generate FIG. 3, wherein 4A and 4B are the acoustic signals (sound pressure and acceleration, respectively) from product A; 4C and 4D are acoustic signals (sound pressure and acceleration, respectively) from product B;
Fig. 5 shows acceleration signals of rubbing on a leave-on product over a time period of 9 minutes, obtained using the system of FIG. 1 wherein two accelerometers and two microphones are used;
Fig. 6. shows acceleration signals of two different products over a certain time period of application, obtained using the same system as that used to generate FIG. 5;
Fig. 7 shows acceleration signals of after-feel of two cleansing products, with measurements taken after a period of towel drying, wherein FIG. 7A shows the dry feel after using a harsh cleansing product; and FIG. 7B shows the moisture feel after using a mild cleansing product.

### Detailed Description of the Invention

Now consumers have been provided with a system and method that meets the need for objective assessment of skin and application and/or wash-off of products onto or from skin.

The present invention is based on a very sensitive method, which detects acoustic signals during touching to assess the in-use sensory performance of personal care products and extract specific sensory attributes or sensory profile, which can be linked to the consumer language and/or communicated to consumers. It can be used to support claims about various benefits from skin care products, such as moisturization, rinsability of cleansers and a wide range of sensory benefits (i.e smoothness, silkiness).

The inventive method can be used:
(1) as a clinical tool to evaluate the efficacy of cosmetic skin care and/or cleansing products, both from a clinical and consumer perspective;
(2) as a consumer communication tool to determine the degree of change that is meaningful and ideal to the consumer, i.e., to define the distribution of skin attributes in a specific population and/or to set technical and consumer targets; or
(3) as a point of purchase diagnostic device to allow a consumer a simple method to evaluate before and after treatment changes in skin condition, thereby supporting claims of product benefits and affecting/influencing product selection, product customization and/or compliance with a product usage regimen.

### ACOUSTIC EMISSION SYSTEM

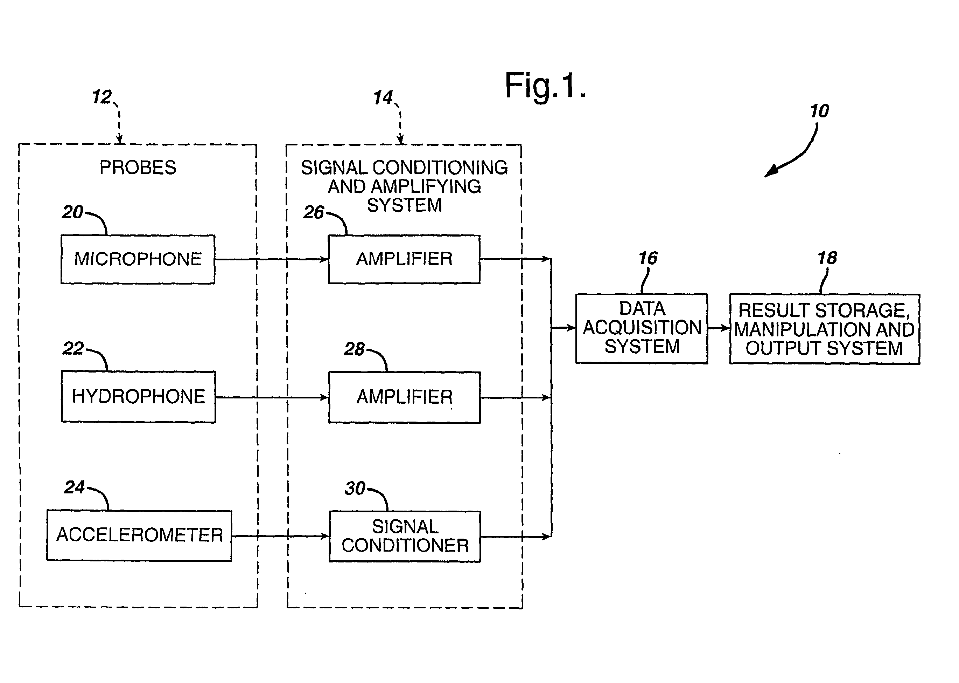

With reference to FIG. 1, a schematic diagram of an acoustic emission system **10** is shown. Acoustic emission system **10** includes probe(s) **12** that operate in an acoustic medium to pick up a signal from sound or vibration generated by a substrate (not shown), such as biological tissues, skin tissues, or hard surfaces. Probe(s) **12** are in communication with a signal conditioning and amplifying system **14,** connected to a data acquisition system **16,** which, in turn, is connected to a result storage, manipulation, and output system **18.** A power source (not shown) is also provided for powering system components. Each of the individual components may be commercially available or custom built, and any of the components **14, 16, 18** may be combined or eliminated, such as, for example if analog signal is stored in that form, or if recording is directly to a tape recorder or CD or DVD.

### Acoustic medium

The acoustic medium may be gas or liquid. Generally, in practice, the acoustic medium is air, water, or aqueous solution.

### Acoustic probe(s)

System **10** has at least one acoustic sensor, or probe **12.** Probe **12** may comprise at least one microphone **20,** for gaseous medium, or at least one hydrophone **22,** for aqueous medium. Microphone **20** and/or hydrophone **22** may be used individually, in combination with each other, and/or in combination with a vibration sensor, or accelerometer **24.** One or more accelerometers **24** may be used individually, in combination with each other, or in combination with at least one microphone **20** and/or hydrophone **22.**

### Signal Conditioning System

Signal conditioning system **14** is in communication with probe(s) **12** and may be comprised of at least one amplifier **26, 28** to amplify sound received by microphone **20** and/or hydrophone **22.** A signal conditioner **30** is provided to manipulate signal received from accelerometer **24.**

### Data acquisition system

The data received by any one or more of amplifiers **26, 28** and/or accelerometer **24** is transferred to data acquisition system **16.** In the alternative, data may be transferred to data acquisition system **16** directly from probe(s) **12.**

### Result storage, manipulation and output system

Data from data acquisition system **16** is transferred to result storage, manipulation and output system **18.** In alternative embodiments, result storage, manipulation and output system **18** may receive data directly from probe(s) **12** and/or signal conditioning system **14.**

System **18** may be a personal computer or similar digital data storage medium, such as a hand-held device (e.g. personal digital assistant [e.g., Palm Pilot^{™}], cellular phone), magnetic tape, CD, or DVD. At this point, the data may be represented in graphical form and printed, in a variety of communication media. Communication media may include the Internet, camera, personal digital assistant (e.g. Palm Pilot^{™}), mobile phone; mobile camera phone, advertising and promotional material, including television, magazines, brochures, posters, flyers, and hand-outs; and/or water-insoluble substrate. Additionally, correlations between skin condition as data received qualitatively from consumers and data received from acoustic emission system 10 may be made and/or may reside within the result storage, manipulation and output system **18.**

System **10** and method can be used to support claims about various benefits from skin care products, such as moisturization, rinsability of cleansers and a wide range of sensory benefits (i.e smoothness, silkiness).

### ACOUSTIC EMISSION METHOD

The inventive method uses acoustic signals generated from contact with skin, when the skin on one area of the human body slides on the skin of another area of the body, i.e., skin on skin. The frictional forces in the skin/skin contact generate vibration patterns that are sensed by probe(s) **12** placed near the skin/skin contact area and recorded by result storage, manipulation, and output system **18.** Advantageously, probe(s) **12** do not affect the contact between one area of skin and another area of skin, since it can detect vibrations near the contact area without interfering with the contact. Therefore according to the present invention, the acoustic signal reflects accurately the skin/skin contact properties and the in-use conditions.

Acoustic emission is recorded during the gentle rub of the hand or finger on another skin part. It is typically detected on the forearm, the hand or the face, but could also be used for other body parts. The acoustic emission signals can be recorded by several kinds of probe(s) **12.** Microphones **20** or hydrophones **22** can be mounted close to the skin/skin contact, but do not touch the skin. For these acoustic sensors, air or water, respectively, is the medium, or the vehicle, of the vibration produced on the skin. Small or miniature vibration sensors (i.e. accelerometers **24)** can be mounted to the skin, near the area of contact. In that case, the vibrations generated by the rubbing are transmitted by the skin tissues. Acoustic sensors may be commercially available devices or custom made devices. The acoustic emission signal is conditioned and/or amplified, by amplifiers **26, 28** and/or signal conditioner **30,** respectively, and sent to data acquisition system **16.** Data from data acquisition system **16** is then sent to storage, manipulation, and output system **18,** such as a PC sound card or other appropriate digitizing device where sound emission data may be saved and displayed. Various standard signal analysis methods can be applied to the signal, and custom methods may be developed.

In typical use, the hand rubs the forearm in a regular manner or the two fingers are rubbed on each other. Since the acoustic emission depends on the speed of the rubbing and the pressure at the skin/skin contact, several recordings are performed for a given subject in order to define a control vibration pattern. The root mean square of the signal recorded over a given period of time can be used to assess the consistency of the rubbing process. The acoustic emission signals are then monitored during the application of products or at intermittent intervals after application of a product.

### Signal Analysis

Applicants have found that the amplitude of the vibrations and the frequency content of the acoustic signal, or waves, monitored as a function of time are very sensitive to the change of the skin/skin contact properties associated with the use of products. The signal wave can be associated with, or correlated with, particular sensory properties. For example, analysis of these signals allows to characterize the rinsability and "feel" or tactile perception of skin cleansers, the tactile feel after use of cleansers, and the tactile feel resulting from the application of cosmetic creams. Acoustic signal wave analysis can also be used to detect the deposition of moisturizing agents from cosmetic products and cleansers.

### COSMETIC PRODUCT SYSTEM

A cosmetic product system is also described including a cosmetic composition associated with acoustic emission system **10.**

A cosmetic product system is described wherein a cosmetic composition is packaged with an acoustic emission system **10** in whole or in part, such as with a typical acoustic graph. A variety of packaging arrangements are envisioned. An acoustic graph may be incorporated as a panel segment of a carton, the latter protectively surrounding the cosmetic composition. In a variation thereof, the graph may be detachably joined to the package through a perforated or weakened construction line, or through an adhesive joinder. In another embodiment, the exterior or interior of the package may be imprinted with instructions for a consumer to sample the product with acoustic emission system **10** located at the point of purchase.

### Cosmetic Compositions

Cosmetic compositions, such as for reducing the appearance of facial skin pores, wrinkles or other undesired facial attributes, may be in the form of creams, lotions, toners, pastes, sticks (e.g. lipsticks), or powders. These cosmetics normally will include a carrier. Suitable carriers include water, emollients (esters, hydrocarbons, silicones, polyols and mixtures thereof), emulsifiers, thickeners and combinations thereof. Most often the carrier will be an emulsion such as an oil-in-water or water-in-oil type. Amounts of the carrier may range from about 1 to about 99.9% by weight.

Pore reduction active or agents for reducing the appearance of pores may include: astringents, humectants, acne and sebum suppressants, desquamation enhancers, keratolytics, and make-up, among other pore reduction actives known to one skilled in the art.

### Astringents

Examples of astringents include, but are not limited to, ethanol, witch hazel, zinc and aluminum salts, and polyphenols.

### Humectants

Humectants include propylene glycol (available from Spectrum), glycerol, and sorbitol, among other humectants known to one skilled in the art. Humectants are known as excellent moisturizers for skin, scalp and hair. See for instance U.S. Patent No. 5,858,340.

### Acne and Sebum suppressants

Anti-acne actives include benzoyl peroxide and salicylic acid, among other anti-acne agents known to one skilled in the art. Sebum suppressants include compounds of the general formula A:

R-O-M (A)

wherein:
R is a branched alkyl or alkenyl chain having at least 7 carbon atoms, and at least two branches;
O is an oxygen atom; and
M is (- (CH₂)ₚO)ₙ- (CH₂)ₘCO₂X)
where n is 0 or an integer between 1 and 7, m is an integer between 1 and 4, p is an integer between 2 and 4; and X is hydrogen, a methyl group, an ethyl group, or a cation. The cation is selected from the group consisting of sodium, lithium, potassium, calcium, copper, magnesium, manganese, strontium, sulfur, zinc, and amine cations. Preferably, X is hydrogen or a cation.

### Make-up

Examples of make-up useful for reducing the appearance of pores include foundations, moisturizers, foamers, and concealers, among other make-ups known to one skilled in the art.

Anti-aging actives may include retinoids, ceramides, alpha or beta-hydroxycarboxylic acids, flavonoids, vitamins, sunscreens, anti-oxidants, preservatives and mixtures thereof.

Typical retinoids include retinol, retinoic acid and retinol esters. The latter include retinyl palmitate, retinyl linoleate, retinyl propionate, retinyl acetate and retinyl salicylate.

Alpha-hydroxy acids include the free acid, lactone and salt forms of glycolic acid, lactic acid, citric acid, gluconolactone, glucarolactone, tartaric acid, malic acid and mixtures thereof. Beta-hydroxycarboxylic acids are exemplified by salicylic acid as well as its esters (e.g. tridecylsalicylate) and salts including ammonium, alkanolammonium and alkalimetal salts.

Ceramides include Ceramide 1, Ceramide 2, Ceramide 3, Ceramide 3a, Ceramide 3b, Ceramide 4, Ceramide 5 and Ceramide 6, as well as pseudoceramides, phytosphingosines and tetraacetyl phytosphingosine.

Other skin benefit agents may be included as optional components. Vitamins may include ascorbic acid as well as its water-soluble and water-insoluble derivatives. Illustrative are ascorbyl tetraisopalmitate, magnesium ascorbyl phosphate and ascorbyl glucoside. Other vitamins include Vitamin B3 (niacin, niacinamide and panthenol), biotin, folic acid, tocopherol and its esters (e.g. tocopherol isopalmitate, tocopherol acetate), Vitamin D and combinations thereof.

Antioxidants include BHT (butylated hydroxytoluene), BHA (butylated hydroxyanisole), disodium EDTA (available from Ciba), sodium citrate, hydroquinone, ferulic acid and esters thereof, green tea extract, lipoic acid, N-acetyl cysteine, resveratrol and combinations thereof.

Amounts of the pore or wrinkle reduction or other actives may range anywhere from 0.0000001 to 30%, preferably from 0.0001 to 15%, more preferably from 0.1 to 5%, by weight.

### METHODS OF USE

The system and method can be used by the consumer directly, but is preferably applied by a beautician or other professional adviser.

Specifically, the system may be used for determining the condition of facial skin pre- and post-treatment, or to track changes in facial attributes, associated with a variety of factors, such as effects of food, activity, menstrual cycle. Pre-treatment acoustic emission system measurements may be used in selecting an appropriate cosmetic product. For example, different product formulations may be recommended depending on the individual skin condition as measured by the inventive acoustic emission system 10 and method. Acoustic measurements may be represented in a variety of media in association with skin care and/or cleansing products within the scope of the present invention.

Subsequent to a baseline analysis of facial attributes using acoustic emission system **10** and method, treatment is begun with a selected cosmetic product for the particular facial attribute. Treatment is continued for a period of time sufficient to allow the product to treat the signs of the particular facial attribute.

After the treatment period of time, such as four weeks, another acoustic measurement is taken. Testing may occur thereafter at 6, 8, 12, 16 and/or 20 weeks. The time intervals and numbers may be longer or shorter. If the cosmetic product is properly functioning, fewer and/or smaller undesirable facial attributes will appear upon acoustic evaluation. This procedure can then be repeated at intervals of six or eight weeks or at any further time interval.

The acoustic emission system **10** and method may be used in conjunction with a variety of media for displaying measurement results, including in or out of home use of the Internet, webcam, personal digital assistant [e.g, Palm Pilot^{™}], mobile phone, and other media capable of displaying the results in graphical, quantitative, and/or qualitative terms. A strip embodying such result may be given out to consumers at point of sale or at a store display.

An objective clinical grading scale, whereby each image is associated with a number, may be developed.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

All parts, percentages and proportions referred to herein and in the appended claims are by weight unless otherwise illustrated.

In the following, several examples of application of the inventive system and method are described. The following is by way of example, not by way of limitation, of the principles of the invention to illustrate the best mode of carrying out the invention.

### EXAMPLE 1

This example uses acoustic emission measurements to characterize the tactile properties on skin of surfactants applied thereto, e.g. slimy, squeaky and slimy/squeaky transition evaluation, using acoustic emission measurements.

An acoustic emission system **10** as generally described with reference to FIG. 1 was used for testing squeakiness and its transition properties of surfactant solutions. One probe **12**, i.e., hydrophone **22** (Bruel & Kjaer, Atlanta, GA, 8103 hydrophone), was mounted onto an inner wall of a container (not shown) for surfactant property testing in aqueous solution. The inner container wall may be machined with certain roughness in order to reduce the reflection of sound waves from the container wall. The charge signal from hydrophone 22 was conditioned to voltage signal via a Bruel & Kjae Conditioner amplifier 28. Clean fingers were rubbed against each other inside the surfactant solution while data acquisition system 16 (CoolEdit 2000 from Syntrillium Software Corporation, Phoenix, AZ) digitized the sound waves into storage, manipulation, and output system 18, i.e., a computer file.

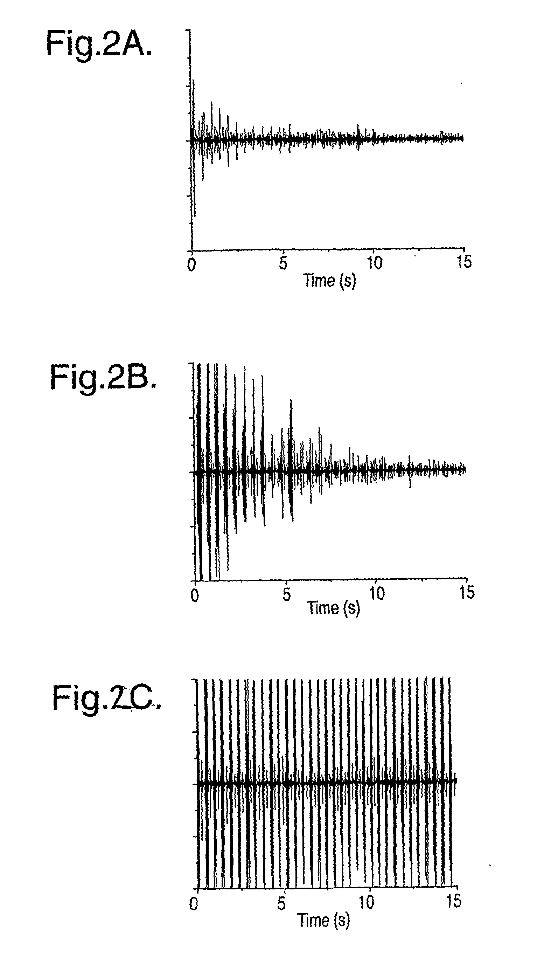

Fig. 2 shows the sound profiles of a common surfactant used in wash-off products, i.e., sodium PEG lauryl ether sulfate (SLES)/water solution at different concentrations, generated using the system of FIG. 1. The observations from these Figures are:
2A 1% SLES/water solution shows very low sound level all the time, indicating a slimy feel or tactile perception;
2B 0.2% SLES/water solution shows the sound level from high to low as finger rubbing time goes on, indicating a transition from squeaky to slimy feel at this concentration;
2C 0.01% SLES/water solution shows a very high sound level all the time, indicating a squeaky feel that does not goes away with time at this concentration.

The differences between these solutions are easily discernible from the results. For higher concentration (e.g. 1% SLES/water solution, Fig. 2A), the SLES film covers the entire skin surface and lubricates the two skin surfaces against their touching. No significant sound can be produced by rubbing fingers (sound pressure is very low). The consumer perceives sliminess of the solution.

With reference to FIG. 2B, when the SLES concentration is lower (e.g. 0.2%), the sound profile is strongly dependent on rubbing time. In the beginning of rubbing, the sound level is very high, corresponding to a "squeaky" feel. As the rubbing continues, the sound level gradually decreases and the consumer can also perceive that the squeakiness decreases. When the solution is perceived as slimy, almost no sound is emitted.

Below a certain SLES concentration (Fig. 2C), the consumer can only feel squeakiness of solution no matter how long rubbing goes.

The results demonstrate a strong connection between the consumer tactile perceptions and acoustic emission signals. Different surfactant systems give different acoustic profiles, thereby representing the different squeakiness/sliminess of those surfactant systems. The acoustic method can be used for quickly and simply evaluating surfactant systems for the tactile perception they produce.

### EXAMPLE 2

This example demonstrates an assessment of the rinse profile of wash-off products, simultaneously using hydrophone **22** and accelerometer **24.**

With reference to FIG. 1, in this example, a typical set-up for assessment of wash-off (cleansing) products was used, employing simultaneously hydrophone **22** and accelerometers **24.** To characterize the rinsability and "feel" of skin cleansers, the acoustic emission signal can be detected by hydrophone 22 immersed in a tank filled with water (not shown) for rinsing an area of skin. Two accelerometers **24** were attached to the subject's skin (PCB 352A24 accelerometer for normal vibration, and PCB 356A15 triaxial accelerometer for tangential and normal vibration). A known amount of cleansers was applied on the wet forearm with the hand of an individual. The arm was then immersed into the rinse tank full of certain hardness water at certain temperature. The other hand rubbed the arm with product while the rubbing sound was picked up by hydrophone **22** and skin vibration was detected by accelerometers **24,** simultaneously.

The acoustic emission signal from hydrophone **22** was conditioned as in Example **1** and recorded by data acquisition system **16.** Signals from accelerometers **24** were conditioned by PCB 442B104 signal conditioner **30** (PCB Piezotronics, Inc., Depew, NY).

Data from data acquisition system **16** was communicated to storage, manipulation, and output system **18,** i.e., a Cool Edit 2000 with sound card digitizing system. In addition, a professional acoustic emission system (Bruel & Kjaer Pulse 6.1 and 7.0, Atlanta, GA) was used.

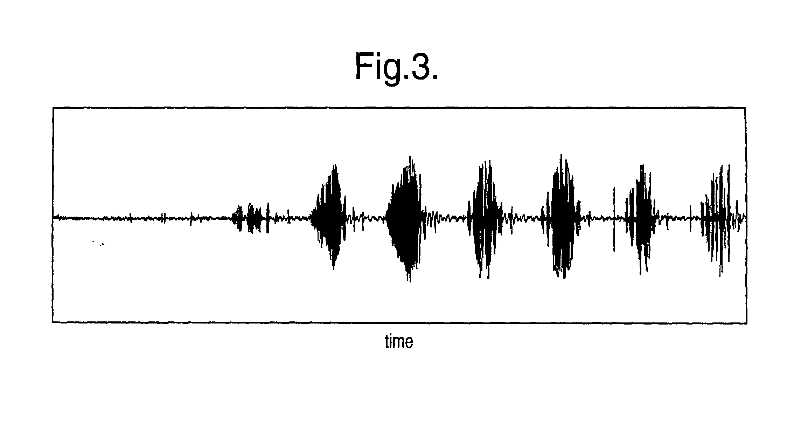

A rinse profile of a wash-off product (TEA-N-Lauroyl L-Glutamate (LT-12)) in soft water (40 PPM, Ca⁺⁺/MG⁺⁺=3/1) thereby obtained is shown in FIG. 3. The figure illustrates how, during rinse, the amplitude of the acoustic emission signal changes significantly. At first, because of the lubricating effect of the surfactants, the acoustic signal is very significantly reduced. The slimy region has no sound or very low sound level while the squeaky region has stronger sound level. As the rinse proceeds, much higher amplitude "squeaky" sounds are produced. The speed of rinse of the cleansers can be assessed on the wave file by measuring the time period before the occurrence of high amplitude squeaky sound. The squeakiness of the cleansers can be assessed by measuring the average amplitude of the squeaky sound. In this particular case, there is no acoustic emission signal from the first three rubs while consumer perceives the sliminess of the wash-off product. From the 4^{th} rub to 10^{th} rub, the acoustic emission signals are different at each rub, indicating the squeakiness changes during rinse. In the 5^{th} and 6^{th} rubs, the more uniform sound profile relates to the smooth feel. In the last several rubs, there are larger interrupts between sound emission spikes, which correlate to the stick/slip of skin surfaces. The consumers usually perceive clean rinse when they reach these rubs.

The whole acoustic profile shows the very rich information during rinse of wash-off products. The different squeakiness can be observed from the sound profile and can be further extracted by applying different data analysis methods (e.g. Fast-Fourier transform (FFT) can reveal the frequency of stick/slip which relates to the squeakiness).

Moreover, the observed acoustic emission profile corresponds extremely well to the consumer's perceptions during rinse.

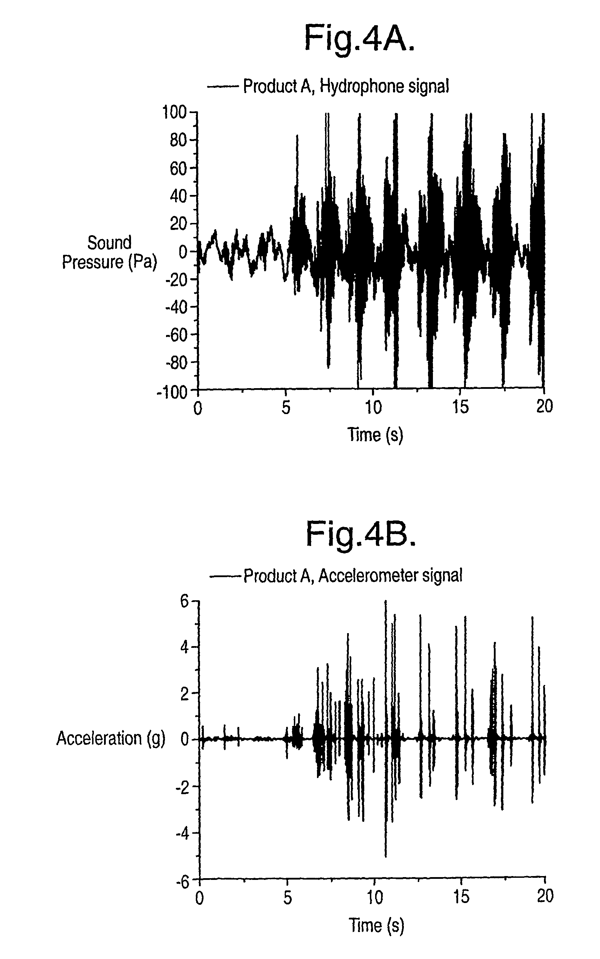

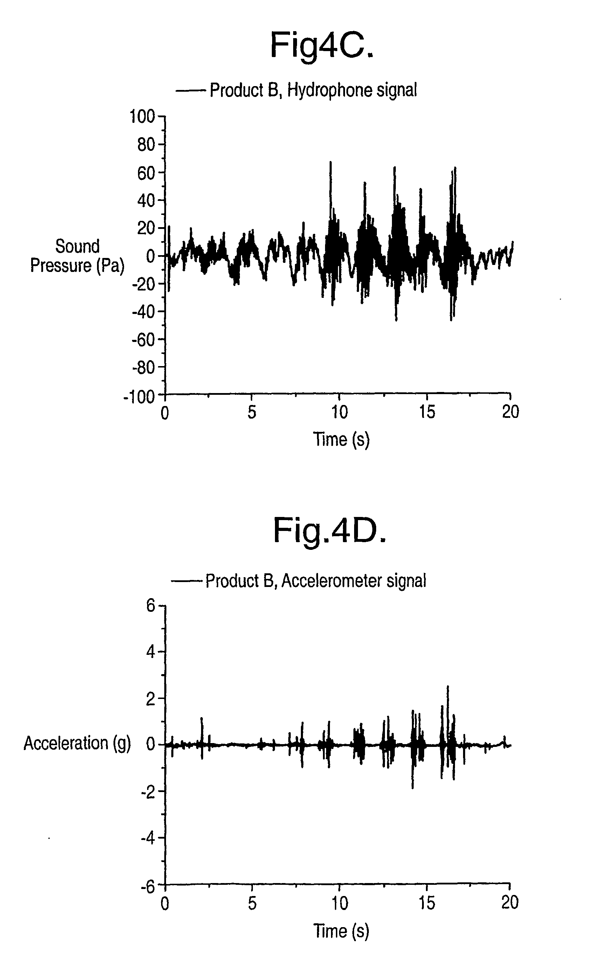

Fig. 4 shows two distinct rinse profiles obtained by rinse in soft water for two different products by acoustic methods using the combination of hydrophone 22 and accelerometers **24** as described in this example. Product A is Kao White^{™} brand bar, a leading soap bar in the Japanese market. Product B is DOVE^{™} brand bar from Nippon Lever, Japan.

Figs. 4A and 4C are signals from hydrophone **22** of two wash-off products. Product A has a quick rinse and feels very squeaky. As shown in Fig. 4A, the slimy region lasts about 5 seconds when the sound pressure is low. In the squeaky region, the sound pressure is higher than 100 Pa. and fine analysis shows the very strong stick/slip signal. For product B, the slimy region lasts about 9 seconds and in the squeaky region, the sound pressure reaches peak value about 60 Pa. But most of signals remain under 45 Pa.

More clear differences between the products can be observed by comparison of skin vibration, as shown in Figs. 4B and 4D. Normal (to the skin surface) vibration of skin surface is shown. The acceleration shown in Fig. 4B can reach 6 g and the frequency of the vibration is medium at the beginning of squeaky region and is very low later, which indicates the strong stick/slip. Japanese consumers usually perceive clean rinse when they reach these rubs, and this it the preferred tactile perception for the Japanese market. However, American consumers perceive this tactile perception as harsh. In Fig. 4D, the acceleration is much lower and the frequency is higher in the squeaky region, which was correlated to a perceived smooth feel.

The combination of hydrophone **22** and accelerometers **24** reveals the consumer perceptions during rinse and can be used as an instrumental tool for conducting consumer tests for wash-off products.

### EXAMPLE 3

This example demonstrates evaluation of sensory properties of skin care leave-on products.

With reference to FIG. 1, system **10** for acoustic measurement and evaluation of sensory properties of leave-on and wash-off products included two accelerometers **24** (PCB 352A24, PCB Piezotronics, Inc., Depew, NY) attached to the subject's forearm. One accelerometer **24** was attached near the palm of the hand and the other near the elbow, to sense the normal vibration of skin surface. Two microphones **20** (1/2" type 4189 pre-polarized free-field microphone, Bruel & Kjaer, Atlanta, GA) were mounted above the two sites where accelerometers **24** were attached. To generate skin vibration, the subject used the other hand (fingers) to rub the forearm starting at the elbow and continuing in the direction of the palm of the hand. Alternatively, a motorized "hand" equipped with loading cell and friction sensor can also be used to rub instead of using human fingers, in order to control the loading, rubbing speed and measure the friction at the same time.

All acoustic emission signals were collected by Pulse data acquisition system **16** (Bruel & Kjaer, Atlanta, GA) and analyzed subsequent to collection by storage, manipulation, and output system **18**.

In the following discussions, we only refer to the signal from accelerometer **24** located closer to the elbow.

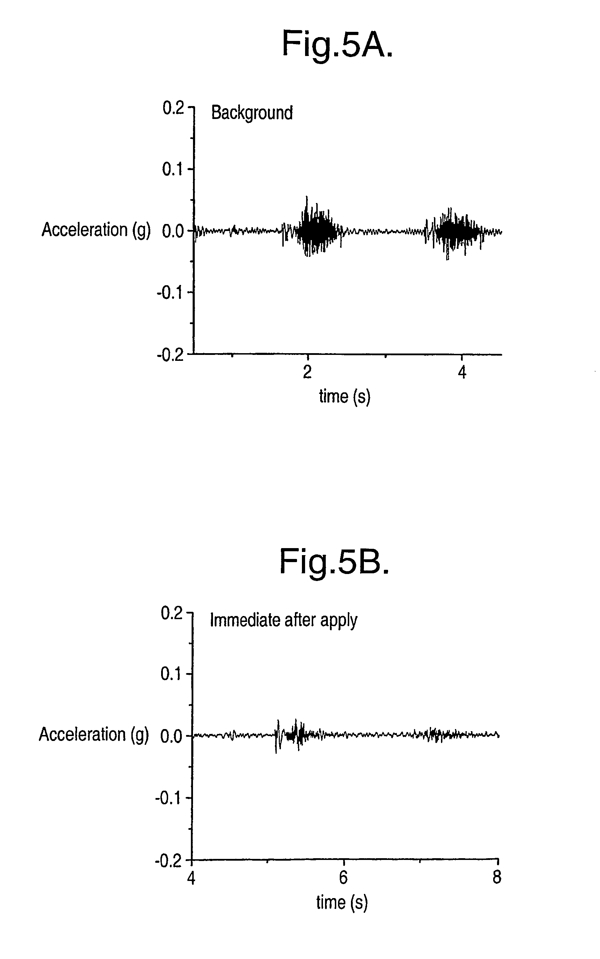

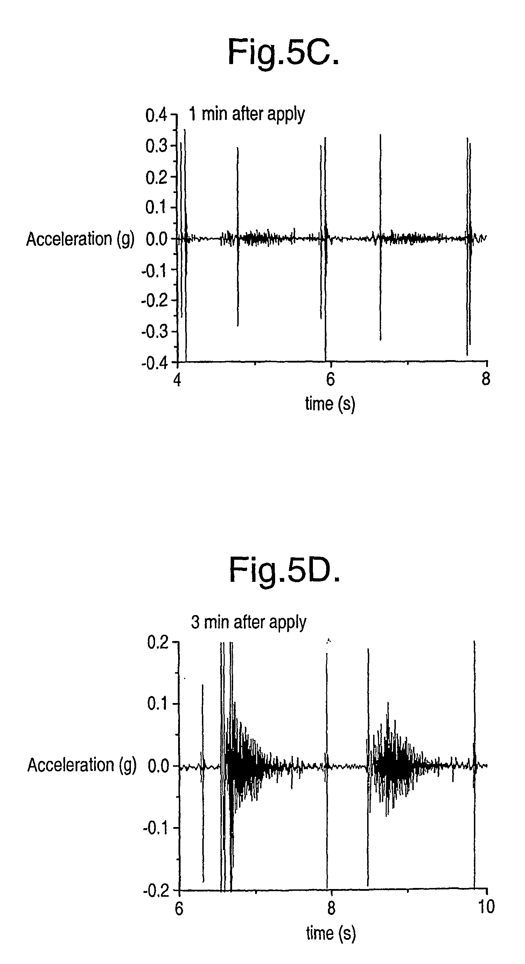

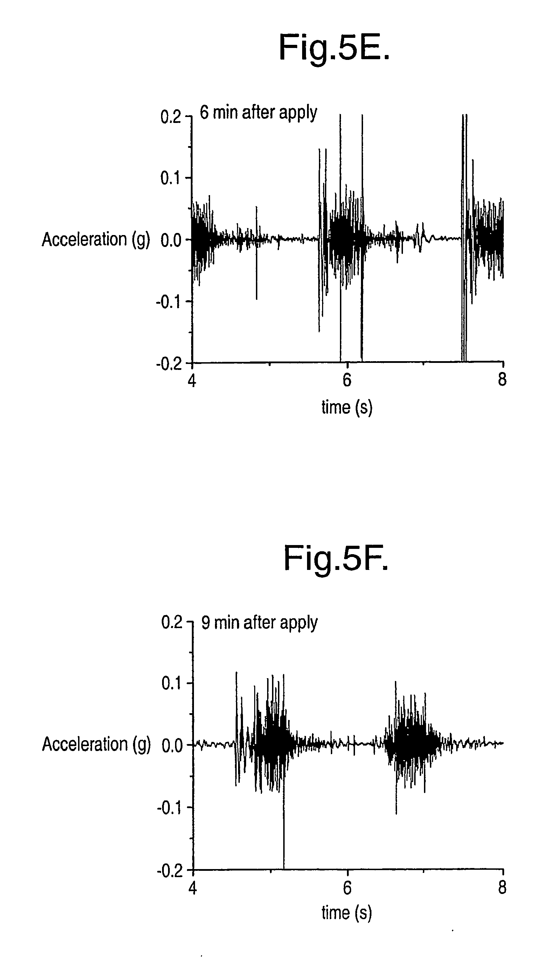

Fig. 5 shows the results of normal acceleration for before and after applying a skin care leave-on product to a subject's forearm. With reference to FIG. 5A, before applying any skin care product, the skin vibrates in a certain band of frequency with certain amplitude. Fair & Lovely^{™} brand lotion, available from Hindustan Lever Ltd, a dry matte product specially formulated for the Indian market preference, was applied to the forearm. It was observed that after application of the skin care product, the skin vibration changed as the product dried. With reference to FIG. 5B, while the product was still wet, the signal from accelerometer **24** showed very low amplitude vibration with lower frequency. With reference to FIGs. 5C - E, during drying of the product, strong vibration peaks were observed. The strong vibration peaks related to the sudden releases of two stretched skin surfaces sticking together during sliding of fingers. At that stage, the subject felt strong dragginess of the product, which is preferred by the Indian consumer and other consumers in hot climates. Fig. 5F shows the acceleration of skin surface after the skin care product dried completely.

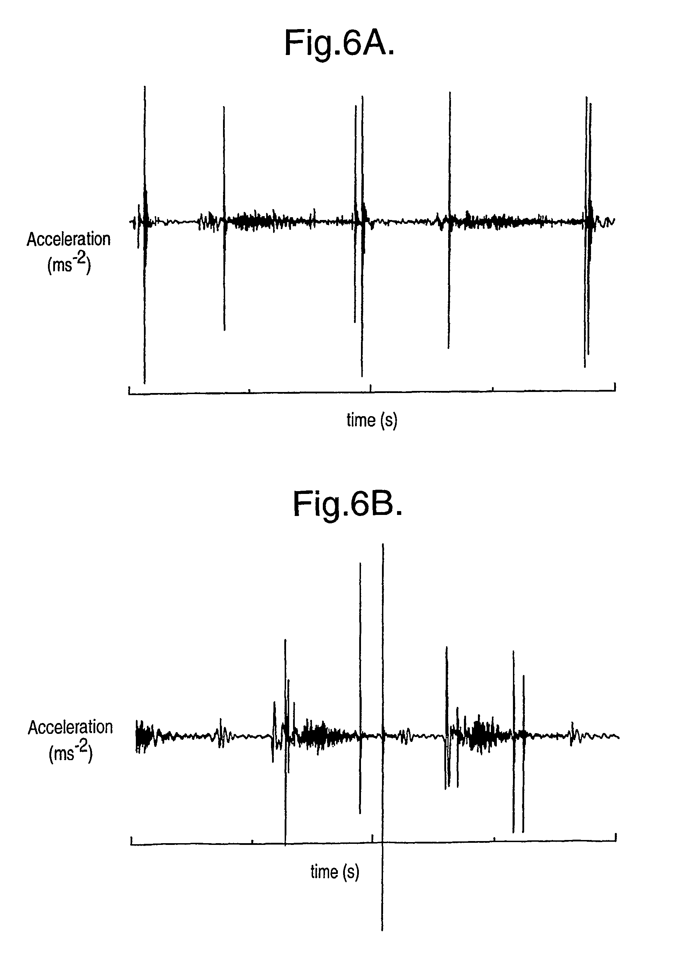

FIG. 6 demonstrates that different leave-on products create different feelings. The vibration detected via accelerometers **24** can differentiate those feelings very sensitively. Fig. 6 shows two acceleration curves of two different products which give consumer different feelings. The signals are displayed at the same scale for easy comparison. Product 1 (data for which is shown in FIG 6A), Fair & Lovely^{™} brand cream, which feels dry and draggy, creates several strong vibration peaks which correlate to the stick-slip event of two sliding surfaces. Product 2 (data for which is shown in FIG 6B), POND'S^{™} brand Age-defying Complex lotion (Chesebrough-Pond's, U.S.) produces an intermediate feel between smooth and draggy. Different consumers may prefer different tactile perceptions.

### EXAMPLE 4

This example demonstrates an assessment of after-use feel for wash-off products using accelerometers **24**.

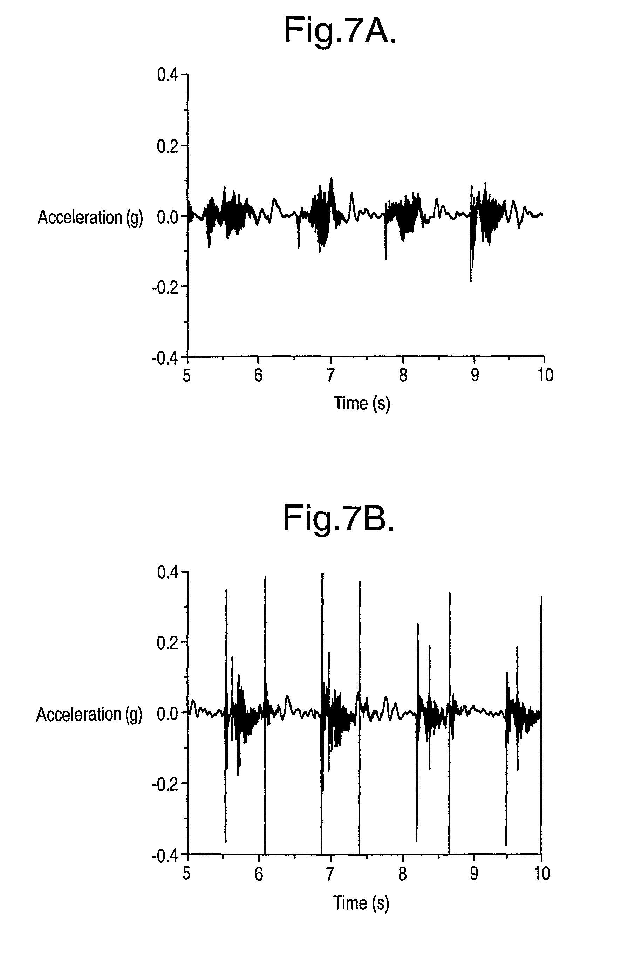

Fig 7 shows two acceleration curves of after-feel. A consumer was asked to use two different cleansing products. After using certain amount of water for washing the products, the skin was patted dry with a tissue. The signals shown here were taken at 2 min after dry. Fig. 7A shows the after-feel curve of Kao White^{™} brand bar, which makes skin more hydrophobic and produces a "dry" feel. Fig. 7B shows the acceleration of a quite different cleansing product, DOVE^{™} brand bar, that makes skin hydrophilic and produces a moisturization feel. The strong stick/slip peaks were observed. The results show that the moisturization agents make the skin more tacky.

### EXAMPLE 5

The present invention is for objectively assessing attributes or condition of an area of human skin. Facial attributes, such as pores, wrinkles and photoaging, may be evaluated. The inventive system and method can be applied for consumer self-evaluation or for evaluation by a beautician or sales associate.

This example illustrates that an evaluation of pre- and post-treatment pore appearance is possible using acoustic emission system **10**, suggesting the validity and usefulness of the system and method of the present invention.

Sheer Coverage^{™} brand foundation, available from Calvin Klein Cosmetics Co., New York, was evaluated using acoustic emission system **10.** Good results were obtained. Consumers perceived a difference acoustic emission system scale, as it correlated well with the visually perceived improvement in appearance of pores after application of the foundation.

### EXAMPLE 6

This example illustrates the use of acoustic emission system **10** and method as a consulting tool at point of purchase and/or as a tool for communicating with consumers.

Generally, consumers in Japan reject for purchase or use soap bars they perceive as "slimy". A new soap bar, based on new technology, is developed and placed on the market in Japan. Acoustic emission system **10** and method are used to communicate to the Japanese consumers:
(a) that the new soap bar has changed tactile perception; and
(b) what will be different for the consumer in terms of sensory and end benefit.

Conversely, the product newly formulated for Japan would now be perceived as having an unpleasant "rub" by the American consumer, and a choice may be made available together with acoustic emission system 10 available at point of purchase to allow the consumer to select the bar that minimizes the unpleasant "rub."

The method can be used as a communication tool to consumers (e.g. advertising).

### EXAMPLE 7

This example illustrates the use of acoustic emission measurement system 10 and method for determining the condition of the skin pre- and post- treatment, and the usefulness of visual improvement in untreated skin condition to induce the consumer to continue using the product.

A consumer took a measurement of the pre-treatment condition of her facial skin using acoustic emission measurement system 10.

Subsequently, the consumer applied a POND'S Dramatic Results^{™} brand product, available from Chesebrough-Pond's, U.S., over a period of about four weeks. Another acoustic emission measurement was taken of clean facial skin without product application. The measurement indicated a significant improvement in appearance of facial skin wrinkles over the period of use.

Although the improvement after four weeks would not have been visually perceptible and would not have been perceptible to the touch, the improvement was evident from acoustic emission measurement, which encouraged the consumer to continue using the POND'S product.

Another advantage of the acoustic emission method of the present invention is that consumers cannot remember the condition of their skin before use. This method gives the consumers a way to compare the difference before and after use of cosmetic products. Therefore, acoustic emission measurements are a good tool for communicating with consumers regarding long term benefits of a given cosmetic product or regimen.

### EXAMPLE 8

Acoustic emission signal expected to result from use of a cleanser as shown in Fig. 2 was printed, folded into a concertina, or pamphlet and placed in a carton also containing the product.

### EXAMPLE 9

This example demonstrates the utility of acoustic emission system **10** and method to define consumer preferences.

During a focus group study, **10** consumers were asked to pick out a skin cleansing product that left them with their "ideal" end point skin after-feel. The product selected by the majority of the consumers was evaluated using the inventive acoustic emission system **10** by the inventive method. With the acoustic emission profile in hand, different product formulations were evaluated to match the one preferred by the consumers.

Thus the system and method served as a tool to generate purchase intent in consumers, as well as a tool for developing products to suit consumer preferences.

### EXAMPLE 10

This example discusses the use of acoustic emission system 10 and method in the development and validation of clinical scale.

In the consumer study discussed above, consumers were also asked to characterize each of the products tested as leaving a "slimy," "smooth," or "squeaky" tactile perception on the skin. This consumer data was correlated with acoustic emission signals for each of the products, to develop an acoustic emission scale that corresponds to consumer perceptions of slimy, smooth, or squeaky. The results of this exercise showed that the two grading methods are highly correlated with one another. The acoustic emission images were thus used as anchors to generate a reproducible clinical scale for the grading of tactile skin perception.

In a separate exercise, two clinicians verified that there was a high correlation between the consumer stated tactile perception and the corresponding acoustic emission signal image.

### EXAMPLE 11

This example demonstrates the utility of acoustic emission measurements according to the present invention in assisting with product selection.

POND'S Institute is set up in Spain, including a vending machine for personalizing leave-on and wash-off cosmetic products.

Preparation of such products immediately upon demand has the advantages of custom products and is particularly advantageous for compositions including unstable ingredients which are best kept unmixed until close to time of use.

In this example, a consumer, with help from a beautician inputs personal preference information for a leave-on product (face cream). Additionally, an acoustic emission measurement was taken to determine the condition of her skin, such as moisture level, skin oiliness, etc.

The vending machine produces a custom cream based on the preference information input by the consumer, as well as based on acoustic emission measurements of her skin condition.

### EXAMPLE 12

This example demonstrates the utility of the acoustic emission system 10 and method for measuring tactile perception of "tacky."

A consumer pushes and lifts fingers to and from a surface which can be described as "sticky," "powdery," etc. The skin vibrates during the pushing and lifting. Acceleration can be measured and used to monitor the feel.

The procedure of this example is particularly advantageous for deodorant products, where "tack" is a very important tactile perception.

### EXAMPLE 13

This example demonstrated the utility of acoustic emission system 10 and method for measuring the cleanliness of hard surfaces. In this example, acoustic emission of skin touching glassware was measured.

The methodology of this example may be used to promote dish cleaning products.

## Claims

1. Use of an acoustic emission measurement system as a clinical tool to evaluate the efficacy of cosmetic skin care and/or cleansing products, the system comprising:
(A) means for detecting an acoustic emission signal generated from skin/skin contact;
(B) means for collecting, storing and displaying said emission signal;
(C) means for correlating said emission signal with an attribute of said skin;
wherein the acoustic emission signal is generated from the skin on one area of the human body sliding on the skin of another area of the body.

2. Use of an acoustic emission measurement system according to claim 1, wherein said attribute of said skin is pores, wrinkles, photoaging, skin texture, or a combination thereof.

3. Use of an acoustic emission system according to claim 1 wherein the means for detecting the acoustic emission signal comprises one or more probes placed near the skin/skin contact area and which sense vibrational patterns generated by the skin/skin contact.

4. Use of an acoustic emission measurement system according to claim 3, wherein the one or more probes do not affect the contact between the one area of skin and the other area of skin.

5. Use of an acoustic emission measurement system according to claim 1, wherein the acoustic emission signal is recorded during the rub of a hand or finger on another skin part.

6. Use of an acoustic emission measurement system according to claim 5, wherein the other skin part is on a forearm, hand or face.

## Patentansprüche

1. Verwendung eines akustischen Emissionsmesssystems als ein klinisches Werkzeug, um die Wirksamkeit von kosmetischen Hautpflege- und/oder -reinigungsprodukten zu evaluieren, wobei das System umfasst:
(A) Mittel zum Detektieren eines akustischen Emissionssignals, das durch Haut/Haut-Kontakt erzeugt wird;
(B) Mittel zum Sammeln, Speichern und Zeigen des Emissionssignals;
(C) Mittel zum Korrelieren des Emissionssignals mit einem Merkmal der Haut;
wobei das akustische Emissionssignal durch die Haut in einem Bereich des menschlichen Körpers, die auf der Haut eines anderen Bereichs des Körpers gleitet, erzeugt wird.

2. Verwendung eines akustischen Emissionsmesssystems gemäß Anspruch 1, wobei das Merkmal der Haut Poren, Falten, Lichtalterung, Hauttextur oder eine Kombination davon ist.

3. Verwendung eines akustischen Emissionsmesssystems gemäß Anspruch 1, wobei das Mittel zum Detektieren des akustischen Emissionssignals eine Sonde oder mehrere Sonden umfasst, die nahe des Haut/Haut-Kontaktbereichs platziert ist/sind und die Schwingungsmuster, erzeugt durch den Haut/Haut-Kontakt, wahrnimmt/wahrnehmen.

4. Verwendung eines akustischen Emissionsmesssystems gemäß Anspruch 3, wobei die eine Sonde oder die mehreren Sonden den Kontakt zwischen dem einen Hautbereich und dem anderen Hautbereich nicht beeinträchtigt/beeinträchtigen.

5. Verwendung eines akustischen Emissionsmesssystems gemäß Anspruch 1, wobei das akustische Emissionssignal während des Reibens einer Hand oder eines Fingers an einem anderen Hautteil aufgezeichnet wird.

6. Verwendung eines akustischen Emissionsmesssystems gemäß Anspruch 5, wobei der andere Hautteil an einem Vorderarm, einer Hand oder dem Gesicht ist.

## Revendications

1. Utilisation d'un système de mesure d'émission acoustique comme outil clinique pour évaluer l'efficacité de produits de nettoyage et/ou de soins pour la peau cosmétiques, le système comprenant :
(A) un moyen pour détecter un signal d'émission acoustique généré à partir d'un contact peau/peau ;
(B) un moyen pour collecter, stocker et afficher ledit signal d'émission ;
(C) un moyen pour mettre en corrélation ledit signal d'émission avec un attribut de ladite peau ;
dans laquelle le signal d'émission acoustique est généré à partir de la peau sur une zone du corps humain glissant sur la peau d'une autre zone du corps.

2. Utilisation d'un système de mesure d'émission acoustique selon la revendication 1, dans laquelle ledit attribut de ladite peau est les pores, les rides, le photo-vieillissement, la texture de la peau ou une combinaison de ceux-ci.

3. Utilisation d'un système de mesure d'émission acoustique selon la revendication 1, dans laquelle le moyen pour détecter le signal d'émission acoustique comprend une ou plusieurs sondes placées à proximité de la zone de contact peau/peau et qui détecte(nt) les motifs de vibration générés par le contact peau/peau.

4. Utilisation d'un système de mesure d'émission acoustique selon la revendication 3, dans laquelle la ou les plusieurs sondes n'affectent pas le contact entre une zone de la peau et une autre zone de la peau.

5. Utilisation d'un système de mesure d'émission acoustique selon la revendication 1, dans laquelle le signal d'émission acoustique est enregistré pendant le frottement d'une main ou d'un doigt sur une autre partie de la peau.

6. Utilisation d'un système de mesure d'émission acoustique selon la revendication 5, dans laquelle l'autre partie de la peau est sur un avant-bras, une main ou le visage.
